# EUROPEAN PATENT APPLICATION

(11) **EP 4 388 981 A1**
(43) Date of publication of application: **26.06.2024**
(21) Application number: 22215771.1
(22) Date of filing: 22.12.2022
(51) Int. Cl.: A61B 5/00, A61B 5/06

(54) **MAGNETO-MECHANICAL RESONATORS WITH REDUCED MUTUAL ATTRACTION**

(71) Applicant: Koninklijke Philips N.V., 5656 AG Eindhoven (NL)
(72) Inventor: GLEICH, Bernhard, Eindhoven (NL); RAHMER, Juergen Erwin, Eindhoven (NL); SCHMALE, Ingo, 5656AG Eindhoven (NL)
(74) Representative: Philips Intellectual Property & Standards

(57) **Abstract**

A system for monitoring and/or diagnosing the transit through the gastro-intestinal tract of a mammal, such as a human is described. The system comprises a micromechanical resonator (MMR) device and a tracking system. The Sensing device comprises a casing and a first magnetic object with a permanent magnetic moment, wherein the first magnetic object is coupled to the casing in such a way, that it is configured for oscillating around an equilibrium position when excited by a magnetic or an electromagnetic excitation field and wherein the tracking system comprises at least one coil for generating the magnetic or electromagnetic excitation field configured for detecting the magnetic response field generated by the Sensing device.

## Description

### FIELD OF THE INVENTION

The invention relates to gastric emptying and device/method for gastric emptying analysis, as well as manufacturing method of such devices.

### BACKGROUND OF THE INVENTION

Gastric emptying is the process by which the contents of the stomach are moved into the duodenum. This is accomplished by three mechanisms: (1) peristaltic waves, (2) systolic contractions of the antrum, and (3) reduction in size of the stomach. To monitor this process, different processes are used.

One of the most widely used method is by using radioactive pills as tracers for monitoring the movement of food. The patient will eat a light meal with radioactive pills (tracers), will lie down on an X-ray table, and the radiologist will make several scans to understand how tracers move in the patient. The tracer will show how food travels through the stomach. This approach has some substantial disadvantages, such as absorbed dose by the X-ray machine and the tracers. Furthermore, the tracers are toxic for the patient and are difficult to handle. Other similar methods also have substantial disadvantages. Barium swallow has the same disadvantages as the radioactive pills. Gastric emptying breath test is imprecise, and the patient will receive substantial dose in the X-ray study. Another known method is to use a smart pill, also known as a wireless motility capsule. The patient will swallow a small electronic device (also known as smart pill, SmartPill by Medtronic, or just SmartPill) and will attach a receiver on the waist. The smart pill will travel through the gastrointestinal system and collect data sent to the receiver. The receiver will then be returned to the physician for reading-out of the data. This method is very uncomfortable to use as the smart pill is quite large, and this might cause gastrointestinal (GI) tract obstruction. Generally, retention of the smart pill is a challenge and additional studies such as esophagogastroduodenoscopy need to be performed, which will cause further discomfort to the patient and will complicate the clinical workflow. Also, the precision of these devices needs to be improved, as in many cases additional studies are ordered to alleviate the problem of insufficient precision. Hence, it is the object of the present application to solve these challenges with a goal to develop a new system and sensor to alleviate the problems of the known methods.

### SUMMARY OF THE INVENTION

The invention is defined by the independent claims. Dependent claims represent beneficial embodiments.

The invention relates to monitoring and/or diagnostic of gastric transit processes in mammals, in particular human beings. Currently, there is no low-cost, easy to handle, absent of harmful side effects for human health (e.g., due to ionizing radiation used in conventional procedures) for gastrointestinal applications. The present invention aims to introduce a solution tackling at least one, and preferably all, of the mentioned challenges.

Very small mechanical devices have been developed, for instance, in the form of microrobots or microdevices, that can be advantageously utilized in applications that introduce a strict size constraint, for instance, in medical applications within the human body. Such microdevices are useful in the form of localization or sensor devices. A very advantageous kind of such microdevices are magneto-mechanical resonator (MMR) devices. A localization or sensing of such magneto-mechanical resonator devices relies on a spatially resolved detection of response signals (e.g., an induced magnetic field) of the magneto-mechanical resonator device which is produced in response to an excitation signal (e.g., an externally applied magnetic field). Information on a location of the magneto-mechanical resonator device is typically obtained by using an array of receiving coils with a known spatial sensitivity profile.

The following application describe the general principles of the Sensing devices and are incorporated here by reference in its entirety as if fully set forth herein: U.S. patent application publication 2020/0397510.

The inventors of the invention have realized that the Sensing devices, such as those described in U.S. patent application 2020/0397510, can be adapted to be used in the gastrointestinal studies and have developed a completely new approach to monitoring and/or diagnostic of gastric emptying, gastric transit processes and generally detection of GI malfunctions in the human body. Since Sensing devices are smaller than the smart pill devices such as the SmartPill from Medtronic, they have the potential of alleviating and/or solving the challenges and problems described previously. Generally, there were no attempts yet to adapt the Sensing devices towards the gastric emptying applications. The inventors of the present invention have developed a completely new approach for monitoring and/or diagnosis of gastric transit processes in mammals based on MMR's. The described approach can also be used in other applications.

The present inventors have appreciated that MMRs devices may be suitable for use as markers and/or sensors (e.g., for measuring temperature, pressure) for gastrointestinal applications, for example, for studies of gastric emptying time. In such applications, it may often be necessary to ingest more than one sensing device at one. As a representative example, the sensing device (100) is a magneto-mechanical resonator (MMR) device. As sensing devices incorporate magnetic materials, the MMRs might attract to each other, which in general is undesirable. Hence, the inventors of the current application have developed a method of using sensing devices for gastrointestinal (and other related applications), as well as a corresponding device configured to measure location, parameters (such as e.g., temperature) of the one or more of the Sensing devices of the present application.

It is therefore an object of some embodiments of the present invention to provide Sensing devices which include features or characteristics which may reduce or minimize their mutual attraction to each other. This is especially useful in context of gastric emptying applications, but this is a non-limiting example, and other possible applications may be envisioned. It is further an object of the present invention to provide such MMRs which are suitable for use in a gastrointestinal tract (GI tract), e.g., configured to pass through the gastrointestinal tract and can be used for making corresponding measurements. It is a further aspect of the present invention to disclose a tracking system configured to measure the signals from the Sensing devices.

According to one aspect of the invention, a system for monitoring and/or diagnosing the transit through the gastro-intestinal tract of a mammal, such as a human is disclosed. The system comprises a micromechanical resonator, MMR, device and a tracking system, wherein the Sensing device comprises a casing and a first magnetic object with a permanent magnetic moment, wherein the first magnetic object is coupled to the casing in such a way, that it is configured for oscillating around an equilibrium position when excited by a magnetic or an electromagnetic excitation field. The device further comprises a Sensing device comprises a second magnetic object configured for providing a restoring force to the first magnetic object, wherein a distance from a center of the first magnetic object to an outer surface of the casing is at least equal to a diameter of the first magnetic object. The tracking system comprises at least one coil for generating the magnetic or electromagnetic excitation field configured for detecting the magnetic response field generated by the Sensing device and a controller for controlling the coil system configured for determining whether the presence of a sensor in a target detection space.

In another aspect, a sensing device for monitoring and/or diagnosing the transit through the gastro-intestinal tract of a mammal, such as a human is described. The device comprises a casing, a first magnetic object with a permanent magnetic moment, wherein the first magnetic object is coupled to the casing in such a way, that it is configured for oscillating around an equilibrium position when excited by a magnetic or an electromagnetic excitation field. The Sensing device comprises a second magnetic object configured for providing a restoring force to the first magnetic object; and wherein a distance from a center of the first magnetic object to an outer surface of the casing is at least equal to a diameter of the first magnetic object.

In some embodiments, as sensing device is described, wherein the distance from the center of the magnetic object to the outer surface of the casing is at least equal to twice, preferably three times of that of the diameter of the magnetic object.

In some embodiments, a sensing device wherein a filling material is provided over an inner side of the casing, wherein the filling material might comprise at least one of: epoxy resin or a polymer material, such as, polypropylene.

In some embodiments, a sensing device has a cage defining an outer shape of the sensor. By "defining" it is meant that the casing of the sensing device is configured to be inside the cage, or in other words, the cage encompasses the sensing device.

In some embodiments, the sensing device is arranged to have a density from 945 to 1155 kg/m3, and preferably from 1000 to 1050 kg/m3. It is preferred to have these densities for the Sensing device to pass through the gastric tract.

In some embodiments, an outer surface of the cage is provided with a gastro-intestinal resistance coating.

In some embodiments the first magnetic object and/or the second magnetic object comprise one or more of the following materials: FeNdB alloy, CoSm, Alnico alloys, Barium ferrites, and/or Barium Strontium ferrites. The first magnetic object may be configured to be attached to the casing via a filament, and the second magnetic object is either configured to be fixed to a casing by a fixing material, and wherein the first magnetic object is configured to freely rotate in response to an externally applied magnetic field or attached to the casing via a second filament.

The ratio of the diameter of filament to the diameter of magnetic object may be a constant, wherein the constant would preferably in the range from 1:1 to 1:1000, and even more preferably from 1:100 to 1: 1000.

In some embodiments, the casing comprises a material, wherein the material is configured to change the morphological structure, such as changing the length of a material under the influence of changing pH levels of an environment in which the device is disposed in.

The Sensing device may comprise a residual magnetic moment and a total magnetic moment, wherein the residual magnetic moment of the device is less than 5% of the total magnetic moment of the device. The residual magnetic moment of the Sensing device is preferably less than 1% and even more preferably less than 0.5% of the total magnetic moment of the device.

In some embodiments, the Sensing device's filament (103) and/or fixing material could be of polymer material group, preferably of class polyepoxides, such as epoxy resin. It is to be understood, that these are merely illustrative examples and other materials of the group, and/or other group of materials can be used.

In some embodiments, the distance between the first magnetic object (102) and the second magnetic object is preferably less than 10%, and even more preferably less than 2% of a diameter of the first magnetic object.

Further, a method of administering the Sensing devices is disclosed. The method comprises providing a plurality of sensing devices of any of the embodiments of the present invention. Administering the Sensing devices for oral consumption by a subject; and
monitoring the sensing devices as they pass through the subject, such as through a gastrointestinal tract of the subject, and configured to be read-out by the tracking system. In some embodiments, the plurality of sensing devices are simultaneously present in the gastrointestinal tract, and wherein at least some of the sensing devices are individually monitored without interference from other one of the sensing devices. Further, the sensing devices may be used for diagnosis in gastrointestinal diseases, and/or for medication adherence control and/or for gastrointestinal constipation monitoring.

In some embodiments, a method of manufacturing of Sensing devices is described, comprising of the following steps:
- providing at least one of the Sensing devices in a fixture;
- manipulating a position and/or orientation of one or both magnetic objects;
- measuring a residual dipole moment of the at least one Sensing device;
- comparing the measured residual dipole moment with a certain predefined threshold, wherein the threshold is preferably 5%, and even more preferably 1%, and even more preferably 0.5% of the total available dipole moment of the Sensing device;
- In case the measured residual dipole moment is greater than the predefined threshold, returning to operation of manipulating the position/orientation of the magnetic objects, and otherwise continuing to the step and optional step, wherein the steps are:
   fixing the position and orientation of the magnetic object;
   and, optionally, applying ultraviolet light to the magnetic objects.

In some embodiments, the method of monitoring the transit through the gastro-intestinal tract of a mammal, such as a human, the method comprising administering the sensing devices as described in the present application the tracking system of any of the present applications are being disclosed.

In some embodiments, a computer program comprising instructions which, when the program is executed by a computer, cause the computer to carry out the steps the method is disclosed. According to one aspect of the invention, a system and/or method for monitoring and/or diagnosing the transit through the gastrointestinal tract of a mammal, in particular a human comprising an ingestible device, such as an sensing device, wherein the sensing device comprises a casing, and a sensor comprising a magnetic object providing a permanent magnetic moment, wherein the sensor is configured to transduce an external magnetic or electromagnetic excitation field into induced mechanical oscillations of the magnetic object, and wherein the induced mechanical oscillations are independent of an external pressure to which the sensor is subjected, and wherein the induced mechanical oscillations generate a magnetic or electromagnetic response field, wherein the distance from a center of the magnetic object to an outer surface of the casing is at least equal to the diameter of the magnetic object.

The systems and methods as described herein are configured for gastric applications, for instance, tracking the ingestible device (-s) in the gastrointestinal (GI) tract of a patient. In some embodiments of the present application, the devices may be of sensing device types. For instance, the sensing devices could be of micro-mechanical resonator (MMR) type.

Often, gastric emptying studies require that multiple Sensing devices are ingested. In the case when multiple Sensing devices are present in stomach, it may be challenging to control whether the individual Sensing devices have come in contact with each other. The Sensing devices that come in contact may interact with each other, which may produce unwanted effects. It is preferably, thus, that the Sensing devices have the interaction limited. Furthermore, it is preferred that the readout system can be used for tracking and adjustment of Sensing device parameters. In this way, the challenges of multiple interacting sensors may be alleviated.

In some embodiments, the outer surface of the casing comprises a material which is suitable for passing through a gastrointestinal tract.

In some embodiments, the distance from the center of the magnetic object to the outer surface of the casing is at least equal to twice the diameter of the magnetic object.

In some embodiments, the distance from the center of the magnetic object to the outer surface of the casing is at least equal to three times the diameter of the magnetic object.

In some embodiments, the casing is cylindrical wherein a diameter of the casing is less than 10 mm.

In some embodiments, the casing is cylindrical wherein a diameter of the casing is less than 5 mm.

In some embodiments, the casing comprises a material which changes length with changes in pH of an environment in which the device is disposed.

In some embodiments, the device further comprises a material layer disposed around a central region of the casing.

In some embodiments, the device further comprises a spacer surrounding at least a portion of the casing.

According to another aspect of the invention, an sensing device comprises: a casing, and a sensor comprising a magnetic object providing a permanent magnetic moment, wherein the sensor is configured to transduce an external magnetic or electromagnetic excitation field into induced mechanical oscillations of the magnetic object, wherein the induced mechanical oscillations are independent of an external pressure to which the sensor is subjected, and wherein the induced mechanical oscillations generate a magnetic or electromagnetic response field, wherein a residual magnetic moment of the device is less than 5% of the total magnetic moment of the device.

In some embodiments, the residual magnetic moment of the sensing device is less than 1% of the total magnetic moment of the device. Residual magnetic moment means a standard definition in the field. A residual magnetic moment describes a moment, wherein the magnetization remaining in a magnetized body is no longer under external magnetic influence.

In some embodiments, the residual magnetic moment of the device is less than 0.5% of the total magnetic moment of the device.

In some embodiments, the device further comprises a second magnetic object. In some embodiments, there may be more than two magnetic objects.

In some embodiments, the second magnetic object is fixed to the casing.

In some embodiments, the distance between the first magnetic object and the second magnetic object is less than 10% of the diameter of the first magnetic object. In some embodiments, this may produce a technical effect of reduce the magnetic field from at least one Sensing device at a position of the other, and reducing higher moments, i.e., not only dipole moments, but generally other moments as well.

In some embodiments, the Sensing device may act as a sensor measuring certain parameters. A non-limiting example may include measuring at least one of: pressure, pH levels, temperature. In some instances, the parameters may be measured by coupling distance to the measured value. This may be done by using a technique described in WO2020253977A1, which is hereby incorporated here by reference. In some embodiments, the distance between the first magnetic object and the second magnetic object is about 2% of the diameter of the first magnetic object.

In some embodiments, the casing comprises a material which changes length with changes in pH of an environment in which the device is disposed.

According to yet another aspect of the invention, a method comprises: providing a plurality of sensing devices; administering the plurality of sensing devices for oral consumption by a subject; and monitoring the sensing devices as they pass through a gastrointestinal tract of the subject. By "plurality" within the context of the present application, at least two Sensing devices are meant. In some embodiment, it may be sufficient to have only one Sensing device to be ingested.

In some embodiments, the sensing devices comprise: a casing, and a sensor comprising a magnetic object providing a permanent magnetic moment, wherein the sensor is configured to transduce an external magnetic or electromagnetic excitation field into induced mechanical oscillations of the magnetic object, wherein the induced mechanical oscillations are independent of an external pressure to which the sensor is subjected, and wherein the induced mechanical oscillations generate a magnetic or electromagnetic response field, wherein the distance from a center of the magnetic object to an outer surface of the casing is at least equal to the diameter of the magnetic object.

In some embodiments, the sensing devices comprise: a casing, and a sensor comprising a magnetic object providing a permanent magnetic moment, wherein the sensor is configured to transduce an external magnetic or electromagnetic excitation field into induced mechanical oscillations of the magnetic object, wherein the induced mechanical oscillations are independent of an external pressure to which the sensor is subjected, and wherein the induced mechanical oscillations generate a magnetic or electromagnetic response field, wherein a residual magnetic moment of the device is less than 5% of the total magnetic moment of the device. The magnetic moment has a certain relationship with respect to the diameter. For instance, taking the cube root of 5% would amount to about 1:3 ratio relationship. Consequently, the radius of the Sensing device could be adjusted by 1/3 to the with respect to a sensing device without the 5% compensation.

In some embodiments, the plurality of sensing devices are simultaneously present in the gastrointestinal tract, and wherein at least some of the sensing devices are individually monitored. In some embodiments, a sensing device can be monitored without interference from other one of the sensing device. In some embodiments, all the Sensing devices can be monitored. In some other embodiments, at least one or several sensing devices can be monitored, and the positioning of other Sensing devices can be deduced based on the information of the location of the measured Sensing devices. As a representative example, one Sensing device may have a low magnetic at the position of the second sensor. The magnetic field from one Sensing device will be close to the other Sensing devices. The Sensing devices may have a coating that would limit the reach of the sensors between each other. In some examples, the magnetic field of individual Sensing devices will be around or below 1 milli Tesla. Preferably, a GI tract resistive coating (i.e., one that would not dissolve under the influence of a GI tract fluids) is used on the Sensing devices 100. The coating used is configured to be non-toxic and not to dissolve in GI tract fluids. In the preferred embodiment, a dip-coating in an epoxy resin may be used. However, other coatings, e.g., spray coating based on acrylics may be used with similar characteristics.

According to yet another aspect of the invention, a plurality of magneto-mechanical resonator (MMR) devices, comprise: a casing, and a sensor comprising a magnetic object providing a permanent magnetic moment, wherein the sensor is configured to transduce an external magnetic or electromagnetic excitation field into induced mechanical oscillations of the magnetic object, wherein the induced mechanical oscillations generate a magnetic or electromagnetic response field, wherein the plurality of sensing devices are present in a gastrointestinal tract of a subject, wherein at least some of the sensing devices are adapted to be individually monitored without interference from magnetic fields or magnetic attraction from other ones of the sensing devices.

In some embodiments, a tracking device for tracking the positioning of the sensing devices is disclosed. The tracking device may be extenral to the sensing devices, such as a a tracking arm comprising specific hardware and/or software for receiving, decoding and analyzing the signals from the Sensing devices. The tracking device may track the position of the Sensing devices from a certain distance. In an examplary example, the Source to Image (SID) distance could be about 2-10 centemeters. However, other SID distances may be envisioned varying to several meters.

The gastric emptying amount can be derived from the food motion and/or volume change. Other approaches for deriving the gastric emptying change can be envisioned within the context of the present invention.

The current sensing device and/or sensing system and corersponding method can be used in mutliple applications for diagnosing of gastrointestinal (GI) problems, such as for diagnosis of abdominal pain, nausea, vomiting and others. This is usually done by performing a gastric emptiying study. The presently described device and methods are applicable for performing such gastric emptiying studies. However, it is to be understood, that the gastric emptying tracking application as described in the present application is merely an example, and the device of the present application can be applied to such, but not limited to, such applications as:
Sensing device tracking in GI tract:
for medication adherence control;
GI constipation monitoring.

The current application has multiple advantages in comparison to the conventional approaches.

One of the advantages, may be that the precision and/or accuracy of the tracking can be improved a high-precision localization algorithm, and improved read-out device.

Another advantage may be that the current invention may mimic the emptying of a normal meal.

Another advantage may be that the radiation exposure can be alleviated.

Another advantage may be that by using the current system, the clustering of several markers due to magnetic force attracting one marker to another can be avoided.

Another advantage may be that the potential independent positions of Sensing devices (e.g., serving as clinical markers) and/or value readings from the devices at different positions can be determined in the e.g., GI tract or other organs of the patient.

Another advantage may be that the safety of the patient is improved since the current devices can operate at sufficiently harmless attraction forces to the patient.

Another advantage may be that the Signal to Noise (SNR) ratio may be improved as the reading distortion is alleviated.

These advantages are exemplary, and other advantages may be envisioned within the context of the present application.
It shall be understood that embodiments of the present invention can also be produced by any combination of the features in dependent claims, or above embodiments, with the respective independent claim.

These and other aspects of the invention will be apparent from and elucidated with reference to the embodiments described hereinafter.

### BRIEF DESCRIPTION OF THE DRAWINGS

Fig. 1 shows a first exemplary embodiment of a sensing device.
Fig. 2 shows a second exemplary embodiment of a sensing device.
Fig. 3 shows a third exemplary embodiment of a sensing device.
Fig. 4 shows a fourth exemplary embodiment of a sensing device.
Fig. 5 shows a fifth exemplary embodiment of a sensing device.
Fig. 6 shows a sixth exemplary embodiment of a sensing device.
Fig. 7 shows a seventh exemplary embodiment of a sensing device.
Fig. 8 illustrates an exemplary method of fabricating a sensing device.
Fig. 9 illustrates an exemplary method of performing a diagnostic procedure using a plurality of sensing devices.. sensing device.
Fig. 10 illustrates an exemplary sensing device with a cage.

### DETAILED DESCRIPTION OF EMBODIMENTS

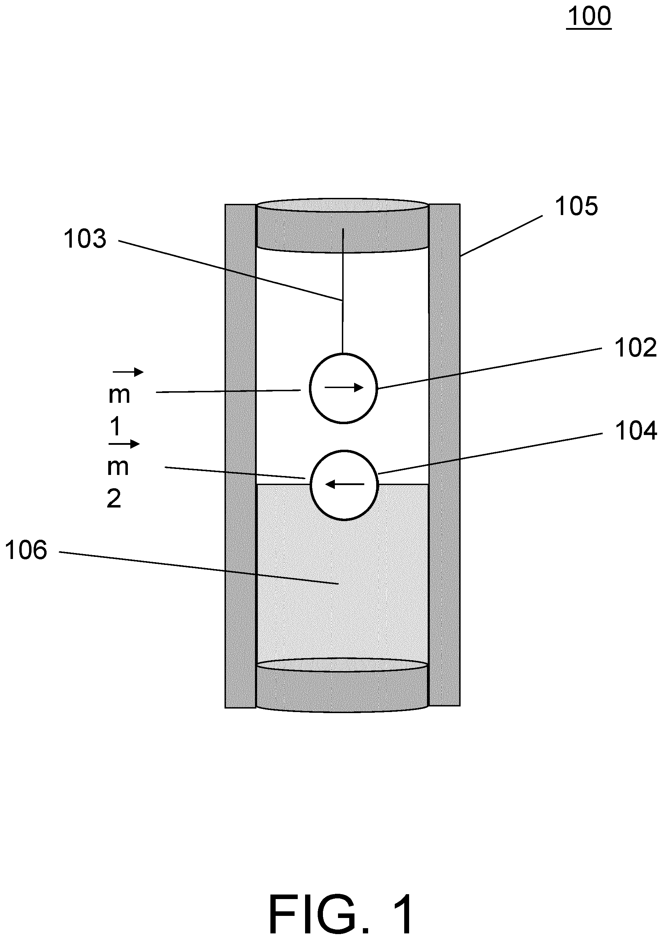

Fig. 1 shows a first exemplary embodiment of sensing device (100). As a representative example, the sensing device is a magneto-mechanical resonator (MMR) device. Sensing device 100 includes a first magnetic object 102 and a second magnetic object 104 disposed inside a casing or housing 105. Beneficially, first magnetic object 102 and second magnetic object 104 comprise permanent magnets. First magnetic object 102 is attached to the casing 105 via a filament 103, and may freely rotate, for example in response to an externally applied magnetic field. Second magnetic object 104 is fixed to casing 105 by a fixing material 106. In some embodiments, the fixing material may be a glue or epoxy. For instance, an epoxy which may be hardened or cured when exposed to light (e.g., ultraviolet light) may be used.

In sensing device 100, the casing 105 is illustrated in the shape of a cylinder. However, in other embodiments the casing may have a different shape, including, but not limited to, a sphere or an ellipsoid.

In some embodiments, magnetic object 102 and magnetic object 104 comprises a FeNdB alloy (neodymium magnet). However, many other suitable materials exist. For example, CoSm may be used, but has some disadvantages such as being more expensive and harder to work with than a FeNdB alloy. Other materials with similar properties, such as Iron platinum, Aluminum Nickel Cobalt (Alnico) alloys, Barium ferrites (BaFe), Barium Strontium ferrites (e.g., BaFe12O19) may be used within the context of the present application. However, the described examples are non-limiting in any possible way and other materials may be used. Beneficially, the magnetic material may possesses a strong remanence while being as light as possible.

In some embodiments, the filament 103 may be made from a high strength polymer material, such as ultra-high-molecular-weight polyethylene (UHMWPE). A polyamide (PA) or any other suitable material with similar characteristics may also be suitable. The length of the filament 103 follows from the dimensions of magnetic objects 102 and 103 and the over-all Sensing device 100. For example, for a magnetic object 102 with a diameter of 500µm, the diameter of the filament 103 may be about 10µm. Beneficially, the ratio of the diameter of the filament 103 to the diameter of magnetic object may approach a constant. The preferred constant would in the range from 1: 1 to 1:1000, preferably 1:50 to 1:1000, and even more preferably from 1:100 to 1:1000.

In operation, magnetic object 102 provides a permanent magnetic moment, and may operate as a sensor, wherein the sensor is configured to transduce an external magnetic or electromagnetic excitation field into induced mechanical oscillations of magnetic object 102. Beneficially, the induced mechanical oscillations are independent of an external pressure to which the sensor is subjected, and the induced mechanical oscillations generate an induced magnetic or electromagnetic response field which may be measured by a tracking system for tracking the location of sensing device 100.

As explained above, the present inventors have appreciated that MMRs such as sensing device 100 may be suitable for use as markers and/or sensors (e.g., for measuring temperature, pressure) for gastrointestinal applications, for example for studies of gastric emptying time. In some embodiments, only the location is measured. In some other embodiments, only the parameters of the body (e.g., temperature, pressure) may be measured. In some embodiments, both the position (localization) and the parameters of the body may be measured.

For gastrointestinal applications, it may be desirable that MMR 100 possess certain characteristics. Some non-limiting examples will be described below.

As one example, it is desirable for gastrointestinal applications that the outer surface of casing 105 comprises a material which is suitable for passing through a gastrointestinal tract. Many suitable materials exist. In general, such materials would not dissolve in the gastrointestinal tract, and beneficially would not be poisonous, but this is not strictly necessary if the material does not dissolve. Beneficially, the material may have regulatory approval for food use. Suitable plastics may include, but not be limited to: polypropylene (PP), low density polyethylene (LDPE), high density polyethylene (HDPE), acrylonitrile butadiene styrene (ABS), polyethylene terephthalate (PET), polytetrafluoroethylene (PTFE), etc. There are also many metals which may be suitable, in particular non-magnetic stainless steel type metals and titanium alloys (e.g., TiA61V4). Cobalt chromium may also be suitable. Many glass and ceramic materials also may be suitable. Generally, all glass and ceramic structures may be suitable, with an exemption of those that contain steel and/or poisonous materials. Aluminum oxides and zirconia may be suitable choices, as may be a vast variety of inorganic glasses. For measuring pH in the gastrointestinal tract, PA or nylon may be preferred for the embodiments of the present application, but other suitable materials may be used, which depends on the envisioned application. For instance, PA may require regulatory approval for this specific application, and other materials may be preferred for some embodiments of the present application.

As another example, sensing device (-s) 100 should be of an appropriate size for passing through the gastrointestinal tract. Beneficially, the diameter of sensing devices 100 may be less than 10 mm. More beneficially, the diameter of sensing devices 100 is less than 5 mm. In some embodiments, the diameter of MMR 100 may be significantly less than 5 mm, for example 1.5 mm or potentially as low as around 0.6 mm. The size of the desired devices depends on the clinical, commercial goals and technical implementation at hand. For instance, having the Sensing devices around 0.6 mm in diameter may be beneficial in some specific applications, but more expensive to produce since more expensive electronics and/or more sophisticated manufacturing steps may be needed. On the other hand, devices of around 5mm in diameter may be more easy and less expensive to manufacture, but they may not be applicable in all of the clinical applications.

As yet another example, it is desirable for gastrointestinal applications that the overall density of sensing device (-s) 100 matches the density of the stomach contents / stomach fluids, which typically is about 1050 kg/m³. Beneficially, the overall density of a sensing device 100 may be from 0.9 to 1.1 times the average density of stomach fluids. In general, this may mean that the overall density of Sensing devices 100 is beneficially in a range from about 945 to 1155 kg/m³. However, other values may be envisioned in different instances. When mass needs to be added to the device to increase its density, it should be added as far out as possible on the oscillation axis of magnetic object 102 to increase the moment of inertia which helps to maintaining a high quality-factor of the sensing device. For example, hull of a casing 105 may comprise a high-density material, such as tungsten, tungsten carbide, tantalum, gold, platinum, iridium, osmium, etc. In other embodiments, a layer of a high-density material may be provided as a ring or belt around the middle region of sensing device 100. In still other embodiments, sensing device 100 may be encased in a spacer material around casing 105 to achieve a desired density for the overall device.

In some embodiments, it may be advantageous to include a sensing feature in the device. For gastric applications, a pH sensitive resonance frequency may be a useful sensor reading. Beneficially, for such an application casing 105 may comprise a material which changes length with changes in pH. For example, a simple polyamide (e.g., nylon) reacts to the pH levels of the environment. Other polymers (e.g., biopolymers like chitosan) may react more strongly to pH levels of the environment. The principle described in this paragraph may be based on the so-called "swelling effect", which is based on a swelling ratio of a certain material when placed in various pH levels of e.g. aqueous solutions. In some instances, this may mean that the Sensing device 100 may incorporate/absorb water and elongate as a result.

In gastrointestinal applications, it may be necessary to ingest more than one Sensing device at once. As sensing devices incorporate magnetic materials, the Sensing devices would be expected to attract each other, which in general is undesirable.

In some embodiments, the magnetic coupling between two or more Sensing devices 100 may be reduced by increasing the distance between the magnetic objects 102 of the Sensing devices 100 when the Sensing devices 100 are disposed next to or near each other. In general, this may be achieved by increasing the ratio of (1) to (2), wherein: (1) the distance from the center of magnetic object 102 to an outer surface of casing 105 to (2) the diameter of magnetic object 102. In some embodiments, the said ratio is at least two. In other embodiments, this ratio is at least three, for example four. In an example embodiment, the ratio of the outer diameter of casing 105 to the diameter of magnetic object 102 is three or greater. In some embodiments, the diameter of magnetic object 102 is about 500 µm and the diameter of casing 105 is about 1.5 mm. In one embodiment, the diameter of magnetic object 102 is at least 200 µm and the diameter of casing 105 is about 600 µm.

One way of accomplishing this is by providing casing 105 with a thick hull. In some embodiments, the hull will be the difference between the actual radius of a first and/or second magnetic object and total sensing device radius, which may be in the range of 2: 1 or 3: 1, or other ratio depending on the application. The hull may also increase the density of sensing device 100, which may be desirable in some embodiments. In some embodiments, a layer of material (e.g., high density material as described above) may be provided as a ring or belt around the middle region of Sensing device 100. In some embodiments, it may be provided as a casing around the Sensing device. In still other embodiments, sensing device 100 may be encased in a spacer material formed around casing 105 to achieve a desired outer diameter. The spacer material for clarity reasons is called a "cage" within the context of the present application.

However, the approaches described above may enlarge the size of the Sensing devices beyond that would be desirable. Many applications call for Sensing devices having a small overall size. Therefore, the inventors have appreciated that other means to reduce the attractive forces between Sensing devices would be highly desirable.

The present inventors have conceived that in applications where multiple Sensing devices may be present in the same vicinity as each other, it would be desirable to have the magnetic fields from an Sensing device decay as fast as possible with the distance from the other Sensing devices. This magnetic fields has two components. The first component is the static magnetic field, i.e., the magnetic field of the undisturbed sensing devices. The second component is the induced magnetic field, i.e., the field which the MMR produces in response to being exposed to an external magnetic field.

The inventors have further conceived that the static magnetic field of a sensing device may be reduced by compensating for the dipole moment of the magnetic objects, and ideally for higher moments, too. The inventors have further conceived that the induced magnetic field of an sensing device may be reduced by making the sensing device magnetically relatively stiff, and therefore the apparent permeability low.

An sensing device may comprise at least two magnetic objects (e.g., 102 and 104), at least one of which can perform a rotational oscillation relative to the other. For a sensing device, in general the magnetic dipole moments of the two objects may be different than each other.

The inventors have conceived that to reduce or minimize the static magnetic field of an Sensing devices, the dipole moments *̅m̅*̅1 and *̅m̅*̅2 should be quite close to each other, not only in magnitude i.e., |*̅m̅*̅1| = |*̅m̅*̅2| but the vectors should, as much as possible, have exactly the opposite direction i.e., *̅m̅*̅1 =-m̅*2.*

However, at small scales, the alignment of the magnetic objects 102 and 104 to achieve this relationship may, as a practical matter, become difficult. The attachment point of filament 103 to oscillating magnetic object 102 may be challenging. Even a 5° misalignment can produce about 10% residual dipole moment of the whole sensing devices 100, where the percentage of residual dipole moment is the overall dipole moment of MMR 100 compared to the dipole moment of one of the magnetic objects 102/104.

This residual dipole moment may be way too large for practical use in some applications.

Therefore, the inventors have devised approaches for reducing this residual dipole moment.

In some embodiments, during assembly of the sensing devices, the residual dipole moment of the sensing devices may be measured, for example by a suitable magnetometer (which might be as simple as small compass needles around the assembly location) or by a proxy method such as the resonance frequency. In the case of sensing devices 100, the fixed magnetic object 104 may be glued or epoxied into place in casing 105 of a sensing device 100 in a fixture that allows adjustment of the angle between magnetic object 102 and magnetic object 104. The angle may be adjusted, for example, by manipulating the position and/or orientation of magnetic object 104, and once the residual dipole moment is less than or equal to a defined maximum threshold, then the glue or epoxy may be cured, for example by exposure to light (e.g., ultraviolet light). In some embodiments, the threshold for the residual dipole moment may be 5% or less of the total available dipole moment (|*m̅*1|+|*m̅*2|) of the MMR. In some embodiments, the threshold for the residual dipole moment may be 0.5% of the total available dipole moment (|m1|+|m2|) of the sensing device 100.

A non-limiting example of adjusting the angle of the Sensing device is described. In some embodiments, the angle of the Sensing device may mean the angle between the magnetic object (104) and the casing (105). The angle may be adjusted in some embodiment, to achieve a desired angle value depending on the clinical application. In some embodiments, this may involve attaching a "stick" (e.g., a piece of wire) and/or manipulating with the "stick" the angle value. This may further involve zeroing the magnetic field. When a desired angle is obtained, the magnet may be glued in place, and at least part of the "stick" may be removed. In this way, the angle of the (movable) magnetic object (102) may follow the (fixed) magnetic object (104) due to magnetic forces.

In other embodiments, instead of identical magnetic objects 102 and 104, one (or both of them) can be composed of two or several sub-objects.

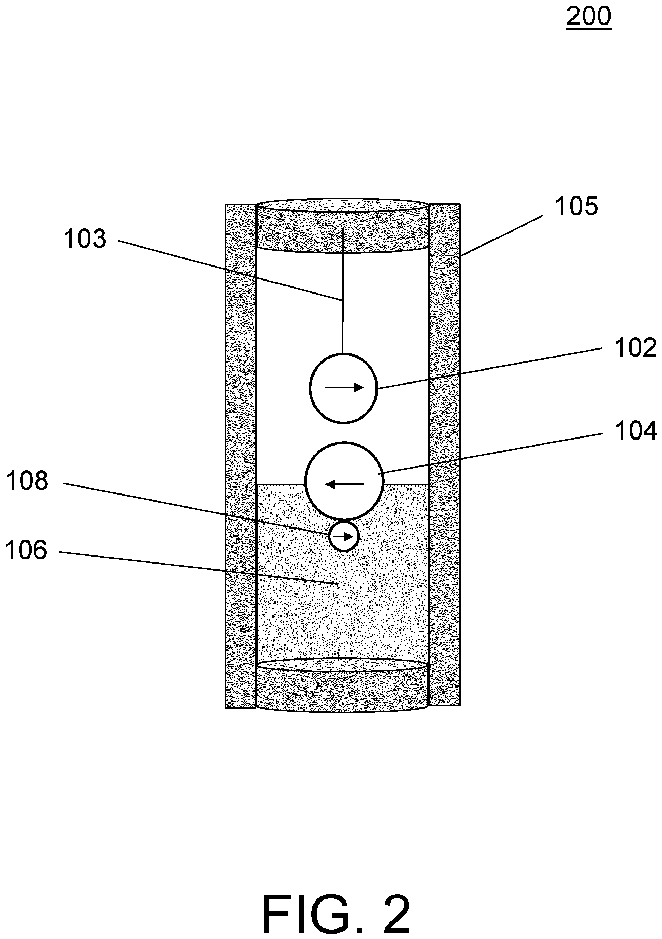

Fig. 2 shows a second exemplary embodiment of a magneto-mechanical resonator (MMR) device 200. A description of elements in MMR 200 which are similar or identical to those in Sensing device 100 will not be repeated. In variation from Sensing device 100, the Sensing device 200 includes an additional, smaller, fixed magnetic object 108 in addition to fixed magnetic object 104. This has the advantage of permitting the dipole moment to be tuned more finely by manipulating a smaller magnetic object. It also allows for a larger fixed magnetic object 104, which will be beneficial for minimizing the induced dipole moment as described below. If properly arranged, a higher multi-pole moment can be reduced, thereby decreasing magnetic field with distance more steeply. Furthermore, although Fig. 2 shows an embodiment with a single smaller magnetic object 708, other embodiments may include two or more smaller magnetic objects.

As mentioned above, it is also desirable to reduce the induced dipole moment of the Sensing devices 100, 200, etc.

In some instances, this may be accomplished by making the torsion spring between the two magnetic objects 102 and 104 stiffer. The torsion spring mainly consists of the magnetic field produced by the fixed magnetic object 104 at the position of the moving magnetic object 102.

There is a minor contribution to the torsion spring from filament 103. It is generally not desired to increase this component, as it tends to reduce the quality factor of the resonance considerably.

Maximizing the magnetic field between the magnetic objects is one way to make the torsion spring between the two magnetic objects stiffer.

In some embodiments, it is preferred to have a substantially spherical or ellipsoidal shape, such as in a form of a pill, for the Sensing device (100). In a non-limiting example, the Sensing device (100) could be a sphere or a spheroid with a minimum diameter of 4mm and a maximum diameter of 8-10mm. Preferably, the Sensing devices can be as small as 0.5 mm to 2 mm in diameter, with a corresponding magnetic sphere diameter between 0.3 mm to 1 mm. The advantage of doing so is to leave sufficient space to have the casing surface far away from the magnetic objects (102, 104) to avoid coupling to external objects, such as other Sensing device. It is preferred, that the outer part of the casing is made stomach compatible, e.g., covered by a material that would be non-soluble in the stomach.

Furthermore, one has to ensure that the sensor is suitable to pass through the gastric tract. This means that the density must be higher than water (to avoid floating in the stomach), preferably between c.a. 950 kg/m³ and 1300 kg/m³. Even more preferably, this would be from 945 to 1155 kg/m³, and even more preferably from 1000 to 1050 kg/m³. This can be achieved by making a standard tracking Sensing device (100) where the casing (105) of the Sensing device acts as the inner surface. The filler material may be provided for achieving a correct shape and size, and the filler material may be coated with a gastric resistant coating (Enteric coating), such as, but not limited to cellulose acetate phthalate (CAP), cellulose acetate trimellitate (CAT), hydroxypropyl methylcellulose phthalate (HPMCP), Shellac. The filler material is chosen such that the Sensing device would have the desired density described above. Alternatively, air is used as filler material, wherein the casing (105) has an inner surface and an outer cage.

Fig. 2, described above, illustrates one way of making this torsion spring greater. In particular, Fig. 2 illustrates the use of a compound fixed magnetic object that creates an even stronger magnetic field between magnetic objects 102 and 104 by increasing the size of the fixed magnetic object 104.

Another way of making the torsion spring between the two magnetic objects stiffer is to bring the two magnetic objects as close to each other as possible.

In some embodiments, a tracking system (100a) for receiving the read-outs from the Sensing devices (100, 200, etc.) is disclosed. The tracking system 100a comprises a housing, which comprises a transmit coil 103a which is connected to a microcontroller 107a via a digital-to-analog converter 107a (DAC) and an audio amplifier 102a for generating the external magnetic or electromagnetic excitation field for Sensing devices (100) which can be embodied as described before. A receive coil 104a is also connected to a microcontroller via a low noise amplifier 105a and an analog-todigital converter 108a (ADC) for reading out the resonance frequency. The microcontroller 107a is connected to a display computer 109a. The microcontroller 107a is configured for, for instance, for signal generation and reception, frequency evaluation and control.

The microcontroller 107a generates transmit pulses that are amplified using the audio amplifier 102a and then passed to the transmit coil 103a which can also be named excitation coil. In this implementation, a separate receive coil 104a is employed, which is decoupled from the transmit coil 103a using two additional decoupling coils 110a. The receive signal is fed into the low-noise amplifier 105a and passed to the ADC 108a of the microcontroller 107a, where a time trace of typically 1/20 of a second is sampled at a rate of about 20 kS/s is deployed. The spacing of the excitation pulses 250 might be continuously adjusted by the microcontroller 107a.

In the embodiments described herein, the tracking system may particularly correspond to a multi-coil system. The use of several coils enables position determination for the marker device by determination of position and orientation of the oscillating magnetic dipole in space. The different amplitudes of the receive signals together with the known coil element sensitivities can be matched to a dipole model for determination of the position and orientation parameters. With many receive coils and channels available, the additional information can also be used for improving background signal suppression as described further below. The several coils may form a multi-coil array which, for instance, may be integrated in a patient bed.

Preferably the coils of the tracking system (100a) are made light enough to be carried around for ease of use. In a non-limiting example, the coils can be made of Aluminium or Aluminium alloys to reduce the weight, which might increase the ease of use. However, in view that housing encompassing the coils needs to be both lightweight and stable/sturdy, composite materials may be used as well. For instance, in some embodiments glass fibre materials may be used. Ideally, all the needed send/receive electronics and power supply are incorporated in a power supply (e.g., LiIon batteries). However, in some application this is not needed, and the device and corresponding coils may be powered by a cable between coil array and electronics.

In some embodiments, the sensing system (100a) is a hand-held sensing device. In order to have a hand-held system, some modifications in comparison to other sensing systems needs to be made. It is beneficial that the system is light enough so that the system can be used ergonomically for analysis of the sensing devices. The inventors of the present application have realized that the sensing system (100a) comprises dedicated components and software for sensing and/or tracking and/or measuring the sensing devices (100) of the present application.

It is to be understood that the tracking system 100a is exemplary and can be applied to any of the Sensing devices 100, 200, 300, etc. of the present application.

In some embodiments, a reference marker may be used on the patients' surface (e.g., placed on the skin) so that the read-out coils do not need to be held while detecting the positions of the Sensing devices 100. As the external marker can be large, it should give a good SNR and can be detected and positioned very rapidly. This marker should be 6 degrees of freedom, DOF, markers (either inherently or a combination of 2 markers of 5DOF, degrees of freedom each) LCQ-Markers, or any other suitable markers. By "degrees of freedom", DOF, a standard definition within the field is meant and can be summarized as: "each of a number of independently variable factors affecting the range of states in which a system may exist, in particular, any of the directions in which independent motion can occur."

It is preferred to have the tracking system 100a and the corresponding coils (transmit coil 103a, receive coil 104a, decoupling coil 110a, etc.) as close as possible to the Sensing devices (100, 200, etc.). In the preferred embodiment, the coils are brought almost in direct contact to the patient, however, with a small gap not to touch the patient's body by the coil elements. Preferably, the distance is no more than 5-10 cm from the patient. However, other distances (e.g., 1 meter between the coils and the Sensing devices may be possible). It may be useful to avoid direct contact as a patient movement would shift the coil assembly and this would need a correction mechanism. For practical reasons, it may be beneficial to have the coil array not touching the patient. However, this is only possible if the signal to noise ratio is high enough and therefore depends on the size of the markers.

The clinical workflow may be as follows. The Sensing devices (100) are ingested according to an approach suitable for a specific clinical application. For instance, for gastric emptying Sensing devices are incorporated in a meal and the patient consumes the meal with Sensing devices 100. For some clinical applications, longitudinal studies may need to be performed. For instance, for a Hinton test replacement, the patient may consume multiple meals over a course of several days, e.g., three times a day for a week.

Preferably, the Sensing devices are read-out at a certain frequency or frequency range. The frequency may generally depend on the final size of the device. For instance, in 1mm diameter Sensing devices, the frequency would be between 500 Hz and 1200Hz. The dependency between the diameter of the Sensing devices and the frequency ranges may have a certain linear relationship. For instance, when the Sensing device would have a 0.5 mm diameter, the frequency range may be between 1000 Hz and 2200 Hz. Other diameters may have other preferred frequency ranges.

After the Sensing devices (100, 200, etc.) are ingested, the location of the markers is recorded. This may be once, several times, or several times a day depending on the clinical goal at hand. For instance, for Hinton test, the measurements are usually done once in the end of the procedure. For gastric emptying, on the other hand, the readout measurements may be performed every quarter of an hour. When the readout measurements have been performed, the number of markers in the different compartments of the human being (e.g., in GI tract) is evaluated. For gastric emptying, this is generally the number of Sensing devices remaining in the stomach. For Hinton test, it may be the total number and the number of sensing devices remaining in the colon.

The skilled person would realize that depending on the clinical goal, the number of measurements, the number of sensing devices consumed, the way they are consumed and the way they are measured might be different. Hence, the examples described here are merely exemplary, and other examples may be envisioned within the context of the present application. The skilled person would also realize that that there are many possibilities to perform the required analysis depending on the clinical goal at hand. However, generally, a distributed ingestion is desired (e.g., at least once a day or 3 times a day). For instance, around 10 sensing devices may be consumed with a single ingestion but depending on the clinical goal this number may vary from 1 to 100. Furthermore, a single read-out event is usually preferred, but depending on the clinical goals, multiple read-outs by the tracking system 200 may be performed.

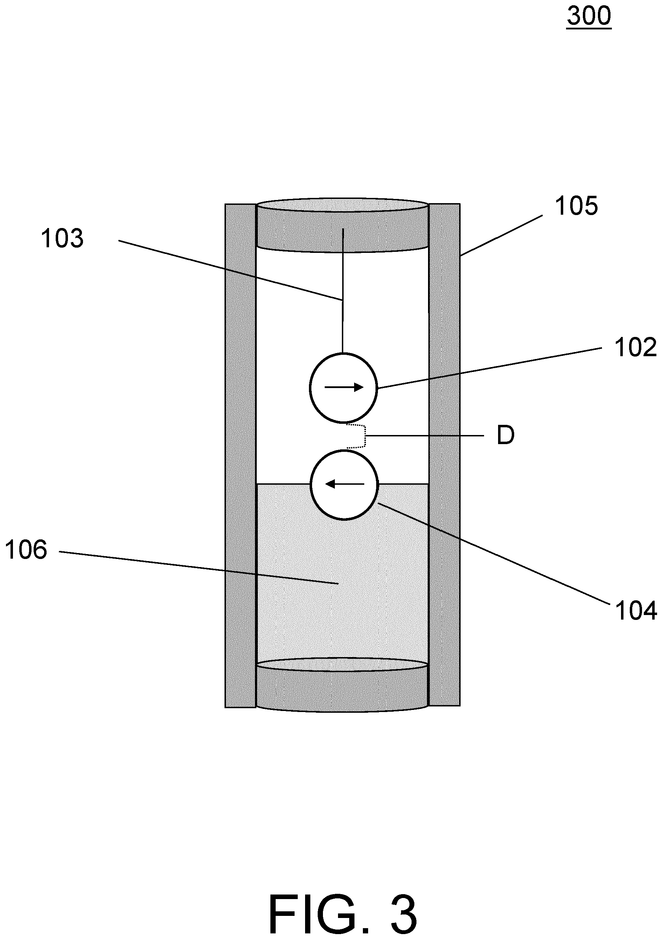

Fig. 3 shows a third exemplary embodiment of a magneto-mechanical resonator (MMR) device 300. MMR 300 is similar to MMR 100, wherein a distance "D" between magnetic objects 102 and 104 has been reduced. For simplicity, the numerals of 100 would be used also for embodiments 200, 300, 400, etc.

The distance D is usually only limited by thermal expansion of the components of Sensing device 100 (e.g., casing 105, filament 103). In some embodiments, at the operating temperature of sensing device 300, D may be about 10% of the diameter of magnetic objects 102 and 104. For example, when the diameter of magnetic objects 102 and 104 is about 500 µm, then D may be about 50 µm. In other embodiments, D may be as little as 2% of the diameter of magnetic objects 102 and 104.

In some embodiments, the magnetic objects 102 and 104 may be in contact at a sufficient low temperature (e.g., 273°K or 32°F or 0°C), but not touching at an operating temperature (e.g., 310°K or 98.6°F or 37°C).

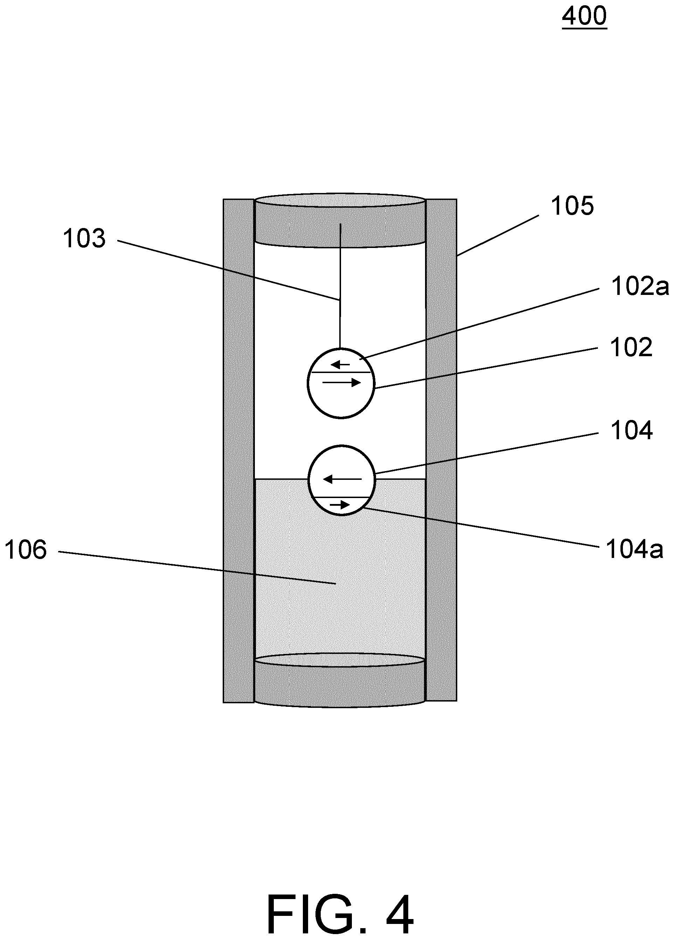

Fig. 4 shows a fourth exemplary embodiment of a magneto-mechanical resonator (MMR) device 400. sensing device 400 is similar to sensing device 100, with a principle difference being that in sensing device 400, magnetic objects 102 and 104 include differently magnetized parts 102a and 104a, respectively, which may reduce or minimize the external magnetic field of MMR 400. This may include exemplary, two different magnets used in each of the magnetized parts 102a and 104a, which may have similar or different properties depending on the clinical need at hand.

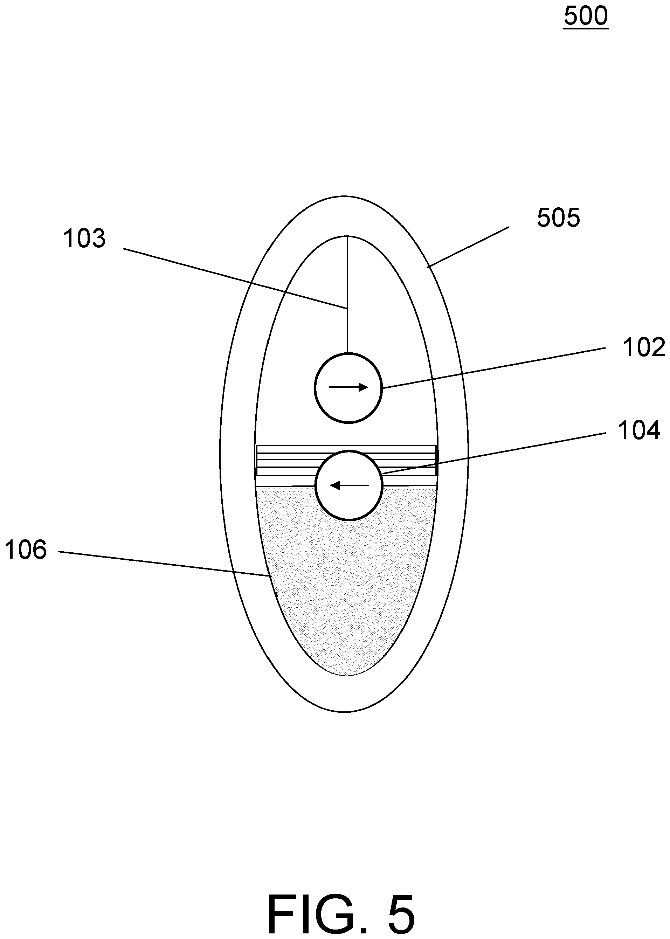

Fig. 5 shows a fifth exemplary embodiment of a sensing device device 500. sensing device 500 is similar to sensing device 100, with a difference being that in sensing device 500, casing 505 has an ellipsoidal shape, rather than a cylindrical one.

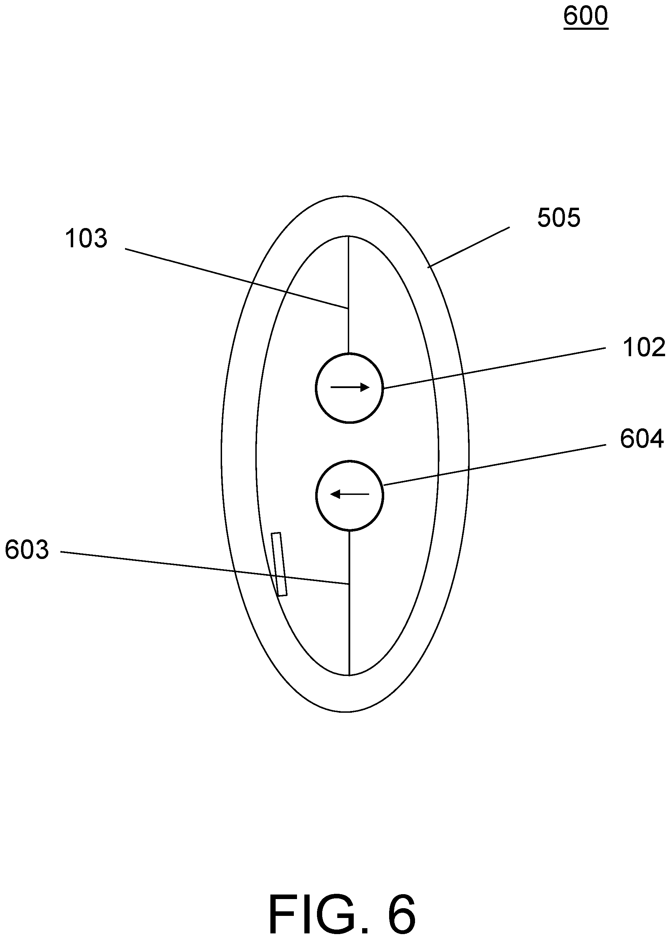

Fig. 6 shows a sixth exemplary embodiment of a magneto-mechanical resonator (MMR) device 600. MMR 600 is similar to sensing device 500, with a difference being that sensing device 600 is a twin-oscillator design where fixed magnetic object 104 is replaced with a second oscillating magnetic object 604 which is connected to casing 605 by a second filament 603.

To further increase the magnetic field between the two magnetic objects in the MMR, in some embodiments the shape of the magnetic objects may deviate from the round shape shown above (which is easy to produce) and instead may take on the shape of a hemisphere, cylinder, or other shaped with substantially flat surfaces which may confront each others upon use. This increases the resonance frequency, which is usually not desired in terms of signal-to noise-ratio (SNR) for the system constraints in gastrointestinal applications. However, the reduction in size of the sensing device may justify the relatively moderate loss in SNR.

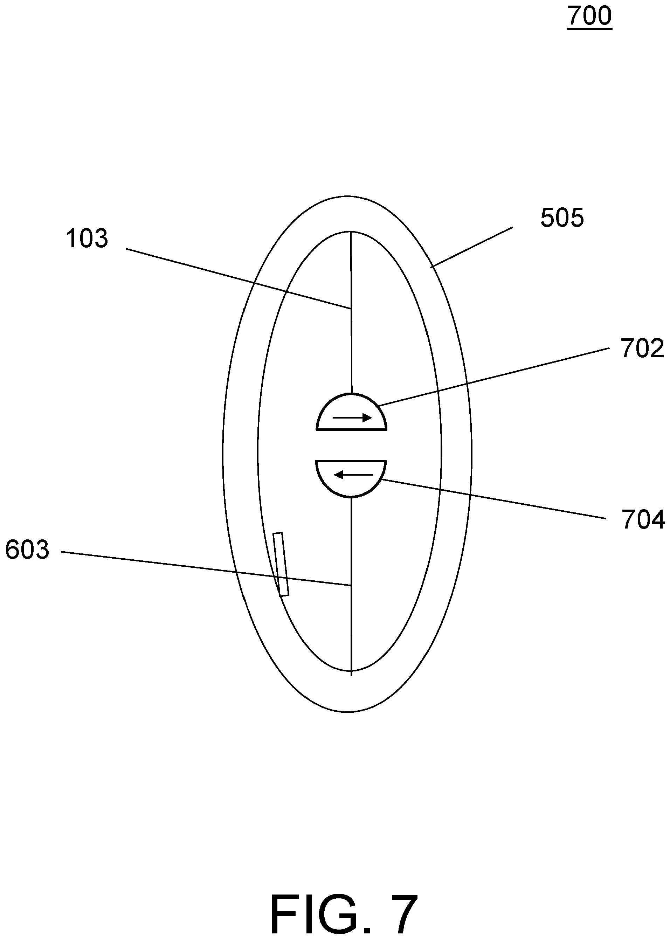

Fig. 7 shows a seventh exemplary embodiment of a sensing device 700.sensing device 700 is similar to sensing device 600, with a difference being that in Sensing device 700, spherical magnetic objects 102 and 104 are replaced by hemispherical magnetic objects 702 and 704.

Ideas, embodiments, and features from Figs. 2-7 can be combined in various ways.

Further, within the context of the present invention, the method administering the sensing devices is described. The exemplary method may be described as follows:
Administering a plurality of magneto-mechanical devices, sensing devices, comprising: providing the plurality of sensing devices;
administering the plurality of sensing devices for oral consumption by a subject; and
monitoring the plurality of sensing devices as they pass through the mammal, such as through a gastrointestinal tract of the mammal,
reading out the plurality of the sensing devices by the tracking system (100a).

In some embodiments, the sensing devices may be used for diagnosis of gastrointestinal diseases, and/or for medication adherence control and/or for gastrointestinal constipation monitoring.

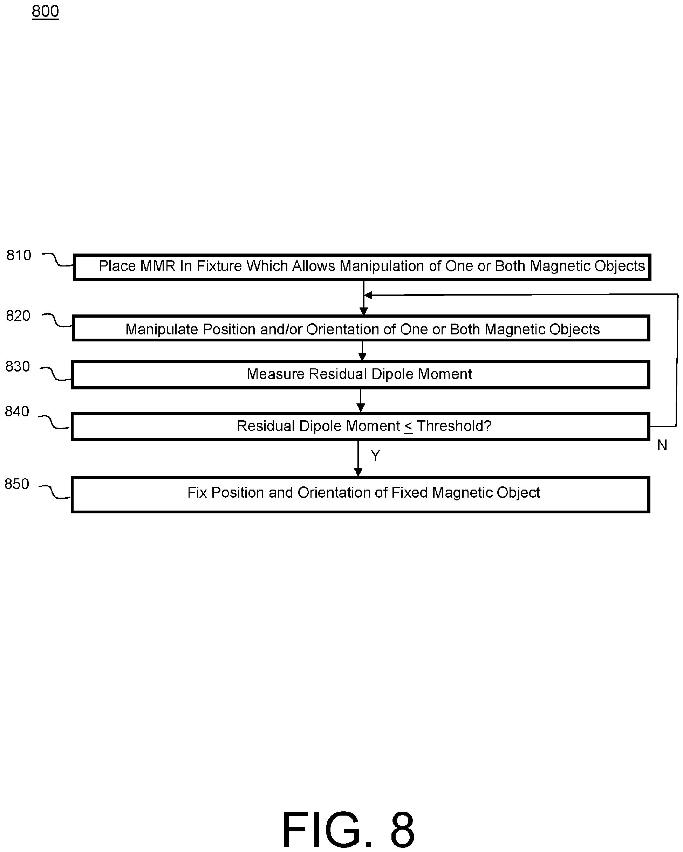

Fig. 8 shows an exemplary method 800 of fabricating a magneto-mechanical resonator, for example magneto-mechanical resonator 100, 200, 300, 400 or 500 as described above.
A method of manufacturing of sensing devices (100, 200, 300, etc.). The method preferably has the following steps:
- providing (810) at least one of the Sensing devices in a fixture;
- manipulating (820) a position and/or orientation of one or both magnetic objects (102, 104);
- measuring (830) a residual dipole moment of the at least one Sensing device;
- comparing (840) the measured residual dipole moment with a certain predefined threshold, wherein the threshold is preferably 5%, and even more preferably 1%, and even more preferably 0.5% of the total available dipole moment of the Sensing device;
- In case the measured residual dipole moment is greater than the predefined threshold, returning to operation 820, and otherwise continuing to the step (850) and optional step (860), wherein the steps (850) and (860) are:
   fixing (850) the position and orientation of the magnetic object (102);
   and, optionally, applying ultraviolet light (860) to the magnetic objects (102, 104).

In some embodiments, some of these steps may be omitted and/or some new steps may be added to the manufacturing method according to the present invention. It is to be understood that this is an exemplary method, and other method steps may be involved. Further, the exemplary method will be described more in-detail.

Method 800 includes a first operation 810 of providing (e.g., placing or mounting) the MMR in a fixture which allows a position and/or orientation of one or both magnetic objects to be manipulated. It is preferred to fixate the Sensing device, in some embodiments, fixture may be avoided.

An operation 820 includes manipulating a position and/or orientation of one or both magnetic objects. In some embodiments, the Sensing device may comprise more than two magnetic objects, and in this way, more than two magnetic objects may be manipulated.

An operation 830 includes measuring a residual dipole moment of the MMR. The residual dipole moment of the MMR may be measured, for example by a suitable magnetometer (which might be as simple as small compass needles around the assembly location) or by a proxy method such as the resonance frequency. In some embodiments, the operations 820 and 830 may be performed by a human. In some other embodiment, these operations may be performed by an automated process executed by a robot, such as an assembly line robot used for production.

In an operation 840, the measured residual dipole moment is compared to a predefined threshold. In some embodiments, the threshold for the residual dipole moment may be 5% or less of the total available dipole moment of the MMR. In some embodiments, the threshold for the residual dipole moment may be significantly less than 5% of the total available dipole moment of the MMR, for example 1% or 0.5% of the total available dipole moment.

If the measured residual dipole moment is greater than the predefined threshold, then the method 800 continues with operation 820, and the position and/or orientation of one or both magnetic objects is manipulated further.

If the measured residual dipole moment is greater than the predefined threshold, then the method 800 proceeds to operation 850 where the position and orientation of the fixed magnetic object is fixed. In some embodiments, this involves curing a glue or epoxy by which the fixed magnetic object is attached to the casing, for example by exposure to light (e.g., ultraviolet light).

As a result of method 800, an MMR may be produced with a residual dipole moment which is 5% or less of the total available dipole moment of the MMR. In some embodiments, the residual dipole moment may be significantly less than 5% of the total available dipole moment of the MMR, for example 1% or 0.5% of the total available dipole moment.

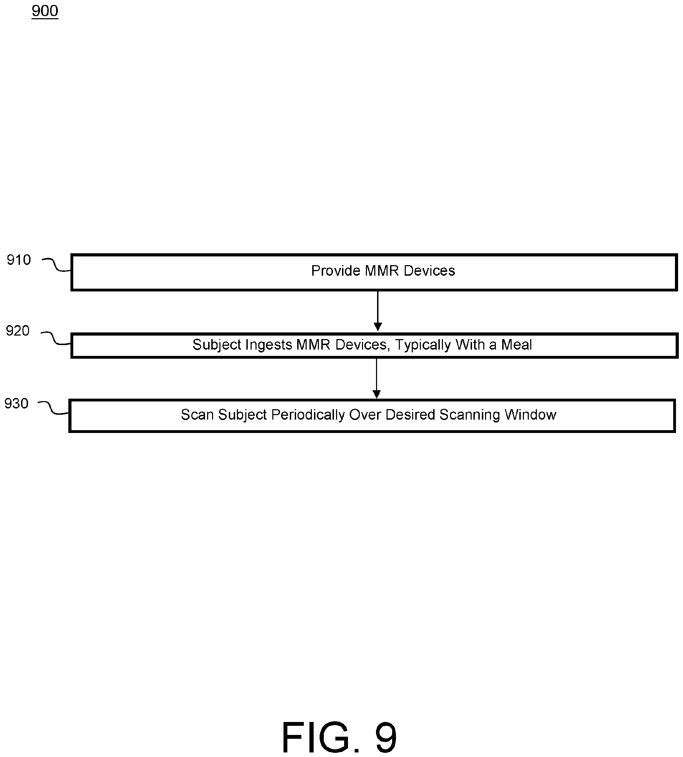

Fig. 9 shows an exemplary method 900 of performing a diagnostic procedure using a plurality of magneto-magnetic resonators.

Operation 910 includes providing a plurality of MMRs. In various embodiments, the MMRs may include one or more of the various MMRs 100, 200, 300, 400, 500, 600 and/or 700 as described above.

Operation 920 includes a subject ingesting the plurality of MMRs. Beneficially, the MMRs may be ingested together with a meal, for example an omelet, white bread, and/or water.

Operation 930 includes scanning the subject, for example the gastrointestinal tract of the subject, periodically over a desired scanning window. In some embodiments, the scanning window may be about 4 to 6 hours, and one scan may be performed with a period of about an hour. It is to be understood, that these numbers are only exemplary, and any desired scanning periods and windows may be employed.

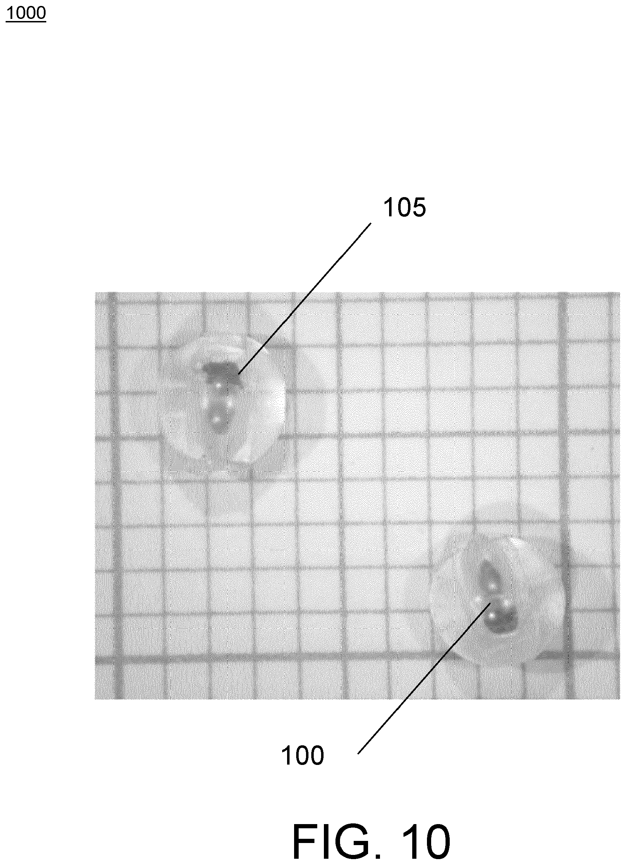

Fig. 10 shows an exemplary an exemplary Sensing device according to the present invention with a cage around it. In the present non-limiting example, the Sensing devices are around 3mm with a cage around the Sensing devices similar to the cage as was previously described. It is to be understood, that the Sensing device may or may not have a cage around them, which depends on the clinical application at hand and/or the desired production process. It is further to be understood that preferably the Sensing devices of the present invention will have a casing (105) around the magnetic objects (102, 104). The casing (105) is essentially a housing that comprises the first and the second magnetic objects (102, 104). It is to be further understood that the cage around the Sensing devices is exemplary, and in some embodiments, it can be omitted. One of the benefits of having a cage around the Sensing devices is to limit the interaction by the Sensing devices between each other, so that the signals read out by the tracking system (100a), are not influenced. Hence, in this way, the magnetic response field generated by the Sensing device can be detected without interference by other Sensing devices.

Furthermore, it is to be understood, that the casing, as was described, previously, has an inner surface, and an outer surface. The outer surface of the casing, i.e., the one facing the GI tract fluids, may be coated with an enteric coating described previously. The inner casing may be covered with materials from e.g., the acryloyl group, such as acryl. It is to be understood that the mentioned materials are exemplary, and other materials may be used for the embodiments of the present application.

In some embodiments, the cage may be soluble in the GI tract, and in other embodiments it may be non-soluble in the GI tract. In the embodiments, where the Sensing devices function also as a sensing device, e.g., measuring at least one of the following parameters: pH, temperature, pressure other parameters, the Sensing devices may measure these parameters within a certain timeframe. This could vary from several minutes to 4 and more hours. In some instances, and in certain clinical applications, it may be beneficial to measure the said parameters when the Sensing device would be close to the rectum, so in certain cases the Sensing devices may be excited (and hence are oscillating) for longer periods of time, such as 24 hours.

With some embodiments of the MMRs described above, it may be possible to administer a plurality of MMRs to a subject and have them simultaneously present in the gastrointestinal tract of the subject without a level of magnetic attraction or magnetic field interference between MMRs which would prevent the MMRs from functioning satisfactorily as intended. Accordingly, some or all of the MMRs may be monitored as they pass through the gastrointestinal tract without interference from magnetic attraction or magnetic fields of the other MMRs.

In some embodiments, to have a low residual dipole moment, it is preferred to use two identical or nearly identical magnets that can be used in e.g., magnetic objects 102 and 104, 102a/104a, etc. It is preferred to use magnets of spherical type that are grinded to produce uniform spheres. However, other implementations may be possible. In an exemplary embodiment, one magnet may be attached to a filament and/or glued to the housing encompassing the Sensing devices (100, 200, etc.) of the present application. The other magnet may be attached to the other end of the housing with a difference that it incorporates a "handle", i.e., a temporarily attached object, such as a wire, that allows the magnet to be rotated in place. In addition, a magnetometer may be placed next to the assembly. In its simplest form, the magnetometer can be a small compass. Then the "handle" may be manipulated until the magnetometer shows the measurements. In some embodiments, it may be preferred to use more than one magnetometer. This is especially preferred in the case of simple compass as it indicates only direction and not strength. However, for more complex magnetometers, this may be alleviated.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

Furthermore, a computer program configured to detect oscillations around an equilibrium position from any of the Sensing devices when excited by a magnetic or an electromagnetic excitation field of a receiving device (100a) is disclosed. The computer program may be run by a general processor, or a specific processor.

Furthermore, a computer readable medium configured to detect oscillations around an equilibrium position from any of the Sensing devices when excited by a magnetic or an electromagnetic excitation field of a receiving device (100a) is disclosed.

It is understood that one or more of the embodiments of the invention may be combined as long as the combined embodiments are not mutually exclusive.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. A computer-readable storage medium can be used.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "sensor" or "system." Furthermore, aspects of the present invention may take the form of a computer program product embodied in one or more computer readable medium(s) having computer executable code embodied thereon. Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. A computer readable signal medium may include a propagated data signal with computer executable code embodied therein, for example, in baseband or as part of a carrier wave.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A system for monitoring and/or diagnosing the transit through the gastro-intestinal tract of a mammal, such as a human, comprising sensing device (100) and a sensing system (100a), wherein the sensing device comprises a casing (105); and
a first magnetic object (102) with a permanent magnetic moment, wherein the first magnetic object is coupled to the casing (105) in such a way, that it is configured for oscillating around an equilibrium position when excited by a magnetic or an electromagnetic excitation field; and
wherein the sensing device comprises a second magnetic object (104) configured for providing a restoring force to the first magnetic object (102);
wherein a distance from a center of the first magnetic object (102) to an outer surface of the casing (105) is at least equal to a diameter of the first magnetic object (102),
and wherein the tracking system (100a) comprises at least one coil for generating the magnetic or electromagnetic excitation field configured for detecting the magnetic response field generated by the Sensing device and a controller for controlling the coil system configured for determining whether the presence of a sensor in a target detection space.

2. A sensing device (100) for monitoring and/or diagnosing the transit through the gastrointestinal tract of a mammal, such as a human comprising: a casing (105);
a first magnetic object (102) with a permanent magnetic moment, wherein the first magnetic object is coupled to the casing (105) in such a way, that it is configured for oscillating around an equilibrium position when excited by a magnetic or an electromagnetic excitation field;
and wherein the sensing device (100) comprises a second magnetic object (104) configured for providing a restoring force to the first magnetic object (102); and
wherein a distance from a center of the first magnetic object (102) to an outer surface of the casing (105) is at least equal to a diameter of the first magnetic object (102).

3. The sensing deviceof claim 2, wherein the distance from the center of the magnetic object (102) to the outer surface of the casing is at least equal to twice, preferably three times of that of the diameter of the magnetic object (102).

4. The Sensing device of any of claims 2 or 3 wherein a filling material is provided over an inner side of the casing, wherein the filling material comprises at least one of: epoxy resin, and/or polymer material group, and/or a material from an acryloyl group.

5. The sensing device of any of claims 2-4 wherein the casing further comprises a cage defining an outer shape of the sensor.

6. The sensing device of any of claims 2-5 wherein the sensing device is arranged to have a density from 945 to 1155 kg/m³, and preferably from 1000 to 1050 kg/m³ to allow the sensor to pass through the gastric tract.

7. The sensor of any of claims 5-6 wherein an outer surface of the cage is provided with a gastro-intestinal resistance coating.

8. The sensing device of any of claims 2-7, wherein at least the first magnetic object (102) is configured to be attached to the casing (105) via a filament (103), and the second magnetic object (104) is either configured to be fixed to a casing by a fixing material (106) or attached to the casing via a second filament.

9. The sensing device of claim 8, wherein the ratio of the diameter of filament (103) to the diameter of magnetic object (102) may be a constant, wherein the constant would preferably in the range from 1:1 to 1: 1000, and even more preferably from 1:100 to 1:1000.

10. The sensing device of any of claims 8 or 9, wherein the filament (103) and/or fixing material is of polymer material group, preferably of class polyoxides.

11. The sensing device of any of claims 2-10, wherein a distance between the first magnetic object (102) and the second magnetic object (104) is preferably less than 10%, and even more preferably less than 2% of a diameter of the first magnetic object (102).

12. A tracking system (100a) for tracking any of the Sensing devices of claims 2-11, wherein the tracking system comprises at least one coil for generating the magnetic or electromagnetic excitation field configured for detecting the magnetic response field generated by the Sensing device and a controller for controlling the coil system configured for determining whether the presence of a sensor in a target detection space.

13. The tracking system according to claim 12, wherein the tracking system is a hand-held tracking device.

14. A method of monitoring the transit through the gastro-intestinal tract of a mammal, such as a human, the method comprising administering the sensing devices of any of claims 2-11, wherein the sensing devices are configured to be read-out by the tracking system of any of claims 12-13.

15. A computer program comprising instructions which, when the program is executed by a computer, cause the computer to carry out the steps the method of claim 14.
